# EUROPEAN PATENT APPLICATION

(11) **EP 3 473 220 A1**
(43) Date of publication of application: **24.04.2019**
(21) Application number: 17812807.0
(22) Date of filing: 13.06.2017
(51) Int. Cl.: A61F 13/15

(54) **REUSABLE COMPRESS**

(30) Priority: 15.06.2016 ES 201630776 U
(71) Applicant: Jimenez Sanchez, Pilar Ignacia, 23740 Andujar Jaen (ES)
(72) Inventor: Jimenez Sanchez, Pilar Ignacia, 23740 Andujar Jaen (ES)
(74) Representative: Bartrina Diaz, José María
(86) International application number: PCT/ES2017/070426
(87) International publication number: WO 2017/216407

(57) **Abstract**

A reusable protective template is formed by multiple layers: a first body-contacting layer made of a textile fabric and having bactericidal properties (silver ions, claim 2) and water-repelling properties due to chitosan fibres; an intermediate layer with silicone or a polymeric gel, and a third layer made of a textile fabric with means of attachment to a garment.

## Description

### OBJECT OF THE INVENTION

The present invention refers to a reusable protective template, for application in feminine garments. Specifically, the protective part of the invention has bactericidal, antifungal, regenerative, healing and hydrophobic properties. It manages to alleviate a disease called bromhidrosis, offering a transpiration of the female intimate area, creating optimum conditions of humidity, disinfection and improving any unpleasant odor.

In addition, since this template is located in the crotch of the female garment, it offers the user protection against friction, including friction caused during physical activity.

Advantageously, the protector of the invention can be reused as many times as necessary, offering the user a way to do without the use of underwear if the textile garment has incorporated the protector of the invention.

### BACKGROUND OF THE INVENTION

Women are four times more likely to get a UTI (urinary tract infection) than men because they have a shorter urethra, which increases the possibility of bacteria reaching the bladder. In addition, the opening of the female urethra is found near the vagina and the anus - places where there are bacteria and the proliferation of infection is more likely.

The type of clothes used, including underwear, are very important in avoiding these infections. These, not being loose, cause humidity as they prevent the entry of air without being able to keep the area around the urethra dry. In fact, if there is a lot of irritation in intimate areas, gynecologists recommend not wearing underwear.

Very tight nylon underwear, lycra, pantyhose, bodysuits, jerseys, etc., trap moisture, thus preventing normal transpiration of the genital area and helping bacteria to grow.

On the other hand, there are currently garments equipped with protectors that cushion a certain area and whose purpose is to increase comfort and reduce the impact on the user during sports, e.g. during the use of a bicycle.

Currently, the existence of reusable hygienic textile protectors is unknown. Specifically, ones that prevent the proliferation of microbes, bacteria and fungi by neutralizing bad odors in the female genital area; and that avoid the rubbing of elastic in underwear or excessive sweating and moisture produced by the simple fact of using close fitting clothing.

### DESCRIPTION OF THE INVENTION

The reusable protective template constituting the invention in itself is a novelty within its field of application, since it is a textile template manufactured, specifically, to prevent infections and discomfort that women suffer in their intimate areas due to exposure to humidity.

The reusable protective template is formed of a plurality of layers, joined to each other. Thus, the template of the invention is composed of:
- A layer of water-repellent technical textile whose structure contains yarns with hydrophobic properties; of fibers incorporating chitin and/or chitosan combined with an active principle, preferably, made from silver ions or zinc oxide.
   Optionally, the chitosan or chitin present in the spinning of the water-repellent technical fabric can be incorporated by means of nanoparticles or microcapsules. Thus, the resulting tissue described gives bactericidal, anti-fungal properties, regenerating and healing, being very suitable to be in contact with the skin and intimate genital area of the user.
   Optionally, the fibers that make up the structure of this technical fabric are natural, synthetic or a combination of these.
- An intermediate layer containing a polymeric gel or a medical silicone having an anti-fungal, anti-bacterial substance.
- A layer of external fabric for fixing inside the garment.

The objective when using the protective template is to avoid the accumulation of moisture, by the presence of waterproof fabrics, allowing perspiration in the area to favor the flow of air. In this way, the template allows the regulation of body temperature in the area, avoiding excess heat and skin irritation and, in addition, eliminating odors generated by sweating. It is even useful as a palliative measure for people who suffer from bromhidrosis, a disease related to bad body odor associated with excessive sweating.

Additionally, the presence of anti-fungal and antibacterial agents contained in the protector of the invention prevents the proliferation of bacteria and fungi and neutralizes odors.

Optionally, the water-repellent textile fabric layer of the insole can contain aloe vera, bamboo, soy proteins, vitamins or algae in order to confer specific benefits or qualities to the template. This according to the desired use and specific benefit intended for the user.

Advantageously, the protector offers the possibility of avoiding the use of underwear when it is adapted to the crotch area of the wearer's garment, providing a physiological feeling of comfort by eliminating possible sources of moisture from the skin, due to its great absorption capacity.

An additional advantage offered by the protective template is its reuse due to the fact that its layers are composed of textile fabrics and polymer gels that maintain their performance after washing, so that in no case will the product be considered disposable.

Additionally, and in order to offer a protector with superior transpiration, the textile and polymer gel layers, previously described, are perforated thus favoring the circulation of air in the area.

The protector may include an additional layer of non-technical textile fabric that will be glued or sewn to the garment. Optionally, when the third layer of the protector is provided with Velcro® or a thermal adhesive, the protector can be easily removed and inserted, without the need for the protector to be always fixed to the garment.

For all the above, the protective insole obtained from the invention offers optimal conditions of moisture and disinfection, which help the perspiration of the skin in that area, so it is considered a highly recommended template for female intimate use and specifically as a product for the prevention and treatment of infections caused by bacteria and fungi.

### DESCRIPTION OF THE DRAWINGS

In order to help provide a better understanding of the characteristics of the invention, a set of drawings is included as an integral part of said description:

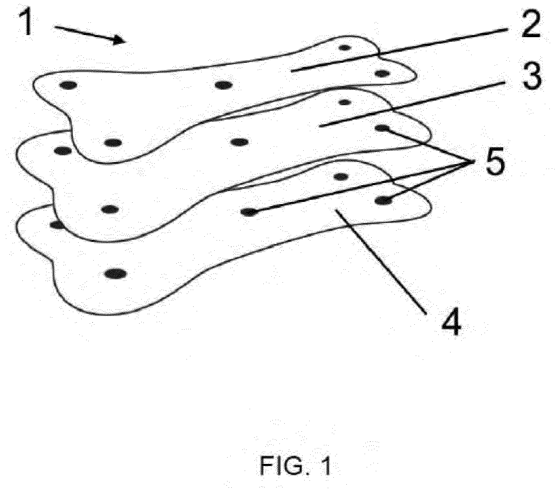

Figure 1.- Shows an explosive representation of the different layers that make up the reusable protective template.

### PREFERRED EMBODIMENT OF THE INVENTION

As can be seen in Figure 1, the protective backing template (1) is composed of three layers that form an indivisible assembly with a preferably elongated shape that allows adaptation to the intimate area of the woman.

Thus, the layer (2) in contact with the skin and the intimate female genital area of the wearer is preferably integrated by an intelligent technical textile fabric composed of a combination of fibers with antibacterial, antimicrobial, regenerating and healing properties, that prevent infections and bad odors and, specifically, that incorporate chitin and/or chitosan.

Optionally, the fabric of the layer (2) can incorporate micro-particles with water-repellent properties that prevent moisture, allowing sweat drops to remain in the template instead of being kept on the skin, or even micro-capsules of zinc oxide and silver ions.

The intermediate layer (3), which lies between the layer (2) in contact with the skin and the layer in contact with the garment (4), is provided with a medical polymer or silicone gel treated with a fungicide to repel the bacteria, fungi, odors, etc.

Advantageously, the surface of the intermediate layer is greater than the layer (2). In this manner, when the layer (2) is positioned centrally on the surface of the intermediate layer (3), a template (1) provided with an outer gel perimeter, integrated by the intermediate layer (3), is obtained. It will be the one that comes into contact with the user's skin, thus offering a feeling of even greater comfort, in the absence of frictions that produce irritations.

Finally, the protector incorporates a layer (4), preferably non-technical textile fabric, which can be sewn or glued with an adhesive to fix the template (1) inside the garment on which it will be positioned.

Additionally, the layer (4) in contact with the garment can be manufactured with Velcro® or similar material by having a completely independent template (1) for use in any garment, becoming a grip layer overlapped to the middle layer (3).

This protective reusable mask (1) is designed to be placed, specifically, in the crotch of the woman and will be as equally washable as the rest of underwear.

Optionally the template incorporates holes (5) to promote the transpiration of the protector.

## Claims

1. Protective reusable template (1) for placement preferably in the crotch area, comprised of:
- A layer (2) of technical textile fabric in contact with the skin of the wearer with bactericidal and anti-fungal properties, whose structure contains threads with hydrophobic properties of fibers with chitosan and/or chitin.
- An intermediate layer (3) containing a polymeric gel or a medical silicone having an anti-fungal and antibacterial substance.
- A layer (4) of textile fabric for fixing inside the garment.
The three layers being joined together.

2. Protective reusable template according to claim 1, **characterized in that** the layer's technical textile fabric (2) in contact with the skin of the user contains an active substance, preferably silver ions or zinc oxide.

3. Reusable protective template, according to claim 1, **characterized in that** the layer's technical textile fabric (2) in contact with the wearer's skin contains chitosan and/or chitin incorporated by means of nanoparticles or microcapsules.

4. Protective reusable template according to claim 1, **characterized in that** the intermediate layer (3) of gel has a surface larger than the layer (2) of intelligent technical textile fabric, providing a template (1) provided with an outer gel perimeter integrated by the intermediate layer (3).

5. Protective reusable template, according to claim 1, **characterized in that** all the layers are perforated, containing holes (5) to facilitate transpiration.

6. Protective reusable template, according to claim 1, **characterized in that** the layer (4) of textile fabric is fixed to the interior of the garment by sewing or with thermal adhesive.

7. Reusable protective template, according to claim 1, **characterized in that** the layer (4) of textile fabric for fixing inside the garment includes a Velcro, or similar to fix the template in an independent manner to the garment.
